# EUROPEAN PATENT SPECIFICATION

(11) **EP 0 725 038 B1**
(45) Date of publication and mention of the grant of the patent: **24.04.2002**
(21) Application number: 96101059.2
(22) Date of filing: 25.01.1996
(51) Int. Cl.: C01G 55/00, C01B 13/00, B01J 23/00

(54) **Material having layered structure of hydrotalcite type, and uses thereof**
Hydrotalcitmaterial mit Schichtstruktur und seine Verwendung
Matériau de type hydrotalcite ayant une structure en couches et son utilisation

(30) Priority: 03.02.1995 IT MI950184
(43) Date of publication of application: 07.08.1996
(73) Proprietor: SNAMPROGETTI S.p.A., 20097 San Donato Milanese (Milano) (IT)
(72) Inventor: Basini, Luca, Milano (IT); Fornasari, Giuseppe, Cremona (IT); Trifiro, Ferruccio, Bologna (IT); Vaccari, Angelo, Bologna (IT)
(74) Representative: De Gregori, Antonella

(56) References cited:
- EP-A- 0 323 192
- EP-A- 0 457 357
- EP-A- 0 640 379
- WO-A-94/14700
- US-A- 5 439 861

## Description

The present invention relates to a material having a layered structure of hydrotalcite type, in which rhodium and/or ruthenium are embedded, and to the uses thereof.

The anionic clays (mixed hydroxides with layered structure, containing, in the spaces between the layers, various kinds of anions -- which may be either exchangeable or non-exchangeable -- and water molecules) are less diffused in nature than cationic clays, but are relatively cheap to prepare in laboratory. One from most studied minerals belonging to this class of compounds is hydrotalcite, the name of which is used in many cases as general reference term (e.g., in such expressions as "hydrotalcite type") for many other isomorphous or polytype compounds. On the other hand, in a large number of other publications, these compounds are also defined as "layered double hydroxides (LDH)".

The large number of useable elements and of variable parameters in the preparation of these compounds, as well as the possibility of providing them with pillars and/or intercalation, make it possible to obtain a large number of advanced materials endowed with specific properties. Therefore, the anionic clays of hydrotalcite type have found many applications as such, or after thermal decomposition in air or in other gas atmospheres.

At present, the main areas of industrial interest are the uses of these compounds as adsorbents, catalysts precursors or carriers, ion exchangers, products for removing acidic residued or water reclamation, flame retardants and/or stabilizers for polymers, as well as applications in medical-pharmaceutical field (as antacids, antipepsin agents and stabilizers or carriers for other drugs). Furthermore, it should be underlined that novel sectors of interest are continuously emerging, such as, e.g. the sectors of production of electrodes or sensors, new semiconductors, defined-thickness carbon films, ferro- or ferrimagnetic materials, and so forth.

In particular, in the literature a large number of examples are reported of use of anionic clays as precursors for multicomponent and/or multifunctional catalysts. As main examples, there can be cited here the preparation of catalyst for methanol synthesis and/or synthesis of methanol and higher alcohols from synthesis gas (Cu/Zn/Co/Al or Cr), basic catalysts for polymerization and/or polycondensation reactions (Mg/Al); for steam reforming of methane and other hydrocarbons (Ni/Mg), photocatalysts and oxidation catalysts (pillared or intercalated anionic clays). The anionic clays have finally been widely used also as carriers for supporting active phases (e.g.: CeO₂, TiCl₄, VCl₄, ZrCl₄, and so forth).

The main properties of the mixed oxides obtained from thermal decomposition of these compounds are: large surface areas, considerable basic characteristics (in particular for Mg/Al systems) and unusual properties to be attributed to the synergistic effects associated with the presence of all of the elements inside the brucitic layers in the structure of the precursor. Furthermore, from the anionic clays homogeneous mixed oxides are obtained which are heat stable and characterized by small size particles, which, by reduction, generate small-size and thermally stable metal crystallites. Finally, the "memory effect" should be mentioned, which make it possible the original hydrotalcitic structure to be reconstructed under mild conditions by bringing the mixed oxides obtained by heat treatment, at not too high temperatures, into contact with aqueous solutions of several anions.

The structure, the properties, the synthesis methods, as well as the industrial applications of anionic clays of hydrotalcite type have been widely and exhaustively illustrated in some recent papers and pubblications [S. Miyata, Kagaku Gijutsushi MOL 15 (10), 32 (1977); W.T. Reichle, CHEMTECH 16, 58 (1986); V.A. Drits, T.N. Sokolova, G.V. Sokolova and V.I. Cherkashiw Clays and Clay Minerals 35, 401 (1987); F. Cavani, F. Trifirò and A. Vaccari, Catalysis Today 11, 173 (1991); A de Roy, C. Forano, K. El Malki and J.P. Besse in "Expanded Clays and Other Microporous Solids", M.L. Occelli and H.E. Robson (Editors), Vol. 2, Reinhold, New York, 1992, p. 108; WO 94/14700; EP-323192; EP-640379).

The application WO94/14700 discloses hydrotalcites compounds having formula

[M²⁺₍₁₋ₓ₎Mₓ³⁺(OH)₂]^{x+}(A_{x/n}ⁿ⁻)·mH₂O,

where M²⁺ is Ni²⁺ or a mixture of Ni²⁺ and at least one other metal ion, preferably having a 2+ valence and more preferably selected from Ca, Co, Cu, Fe, Mg, Pd, Pt, Sr, Ti, V Zn and Zr²⁺, M³⁺ is at least one metal ion having a valence of 3+ preferably selected from Al, B, Cr, Fe, Ga, In, La, Ni, Co, Mn, Rh, Ti, Tl and V, the anion A can be any anion that provides for a hydrotalcite-like structure (for example carbonate, nitrate, halide, chlorate, sulfate,hydroxide,oxide, carboxylates, polycarboxylates).

The application EP-323192 discloses hydrotalcites having the formula

Mg_{4.5}(Al₂Ru_{2/3})(OH)₁₅ (Cl)₂·3.5H₂O

or

Mg_{4.5}Al₂(OH)₁₃(Cl)₂·3.5H₂O 2/3Ru(OH)₃

where the Ru ions, adsorbed or supported, do not enter in the crystalline structure of the hydrotalcite but they remain outside.

The application EP-640379 discoses hydrotalcites having the formula

MₘNₙ(OH)₍₂ₘ₊₂ₙ₎Aₐ·bH₂O,

where M is a divalent metal cation selected from Co, Cu, Ni, Pd, Zn, Fe, Mn and Mg, N is a trivalent metal cation selected from Al, Ga, In, Fe, Rh, Co, B, Cr, La, Sc, Y and rare earth metals, A is a mono-, di- or tri-valent anion, b is an integer having values of 1 to 10.

It also specifies:

MₘAl³⁺ ₙ(OH)₍₂ₘ₊₂ₙ)Aₐ · bH₂O

wherein
- M: is Co²⁺, Cu²⁺, Ni²⁺, Pd²⁺, Mg²⁺ or mixtures thereof;
- A: is a mono-, di- or tri-valent anion which decomposes when heated to a temperature sufficient to form a volatile gas;
- m and n: are such that m/n has values of 0.5 to about 6;
- a: is a number with the provisos that when A is a divalent anion, a = 1/2n and when A is a trivalent anion a = 1/3n and
- b: is an integer having values of 1 to 10.

Optionally, a portion of the aluminum atoms can be replaced by Cr, V, La, Rh and Fe to yield active catalysts for N₂O decomposition.

A specific example with Co/Rh/Al is disclosed.

We have now surprisingly obtained a material having a layered structure of hydrotalcite type in which rhodium and/or ruthenium are inside the interior of said structure, which potentially can have a large number of industrial applications, in particular as catalyst or catalyst precursor.

The material according to the present invention, having a layered structure of hydrotalcite type, is characterized in that rhodium and/or ruthenium are contained inside the interior of the structure having the following general formula:

[RhₐRu_{b}X_{c}Y_{d}(OH)₂]^{z+}(Aⁿ⁻ _{z/n}).mH₂O,

wherein
- X: is a divalent or univalent metal cation,
- Y: is a trivalent or tetravalent metal cation,
0≤a≤0.5,
0≤b≤0.5,
0.5≤c≤0.9,
0≤d≤0.5,
a+b+c+d=1,
- m: is 0, or represents a positive integer,
- A: can be a hydroxyl, any inorganic, organic anion, iso- or hetero-polyanion, anionic complex or organometallic complex, having "n" electrical charge,
- Z: is the total electrical charge of the cationic component, with the exclusion of the value φ for the addition a+b.

The atomic ratios of the elements are preferably comprised within the following ranges:
0≤a≤0.33,
0≤b≤0.33,
0.66≤c≤0.8,
0≤d≤0.33.

The divalent metal cation X is preferably selected from Mg, Ni, Co, Zn, Fe, Mn, Cu, Ca and Cd.

The univalent metal cation X is preferably lithium.

The trivalent metal cation Y is preferably selected from Al, Ga, Ni, Co, Fe, Mn, Cr, V, Ti, and In.

The tetravalent metal cation Y is preferably titanium.

The preparation of the material according to the present invention can be carried out analogously to as disclosed in "Catalysis Today" and in "Expanded Clays and Other Microporous Solids" (already cited above).

In particular, in the examples reported in the following, the catalytic materials obtained were prepared by using an aqueous solution containing, in the suitable proportions, the salts of rhodium and/or ruthenium, of a divalent or univalent element, and of another, trivalent, or also tetravalent, element. This solution was added, with strong stirring, and at a temperature comprised within the range of from 30 to 90°C, and preferably of from 50 to 70°C, to a solution of alkali-metal carbonate or hydrogencarbonate, caring, also by means of the addition of acids or bases, that throughout the precipitation the pH would remain at a constant value comprised within the range of from 7 to 12, and, preferably, 8 to 11. In such a way, the simultaneous precipitation of all of the elements and a fine interdispersion thereof was accomplished. The resulting crystalline precipitate was separated and washed with, preferably hot, water, until a content of alkali metal, expressed as oxide, of less than 0.1% was reached. The precipitate was subsequently dried at 100°C and calcined in air or nitrogen at a temperature comprised within the range of from 200 to 1,100°C, preferably 350 to 950°C.

The material according to the present invention is particularly active in the reaction of partial catalytic oxidation of natural gas to obtain synthesis gas. The process can be carried out under high space velocity conditions, at significantly lower temperatures than as presently used, and with even particularly low molar ratio of CH₄:O₂ and CH₄:H₂O.

The reaction is carried out by operating with the catalyst according to the present invention in the form of a stationary bed, to which a gas stream is fed which contains methane and oxygen, possibly diluted with an inert gas, with a molar ratio of methane to oxygen, CH₄:O₂, higher than 1.5, and under pressures comprised within the range of from 50.6 KPa (0.5 atm) to 5066.2 KPa (50 atm), preferably of from 101.3 KPa (1 atm) to 4053 KPa (40 atm). The process can be furthermore carried out at a temperature generally comprised within the range of from 300 to 1,000°C, preferably of from 350 to 950°C, and with very high values of space velocity generally comprised within the range of from 20,000 to 1,500,000 h⁻¹, preferably of from 100,000 to 800,000 h⁻¹.

By operating with rhodium or ruthenium containing material according to the present invention, very high values of methane conversion and selectivity of converted methane into carbon monoxide and hydrogen are obtained.

The use of the material claimed in the instant patent application as catalyst makes it possible to achieve a greater simplicity and a higher reproducibility of the preparation method as compared, e.g., with those presently used catalysts which require an impregnation step.

Further details are seen in the claims.

Furthermore, by suitably selecting the composition, the catalytic activity can be modified and/or regulated.

The rhodium or ruthenium containing material according to the present invention can also be used in the reactions of reduction of nitrogen oxides, of hydroformylation, of hydrogenation of CO, CO₂ and mixtures thereof, of water gas shift, of hydrogenation of organic substrates in liquid or vapour phase (such as anhydrides, benzene, cyclohexene, alkenes and alkines, which may be either halogenated or not, oximes, aromatic or aliphatic nitrocompounds, aromatic or aliphatic ketones, aromatic or unsaturated aliphatic amines).

Some examples are now supplied in order to better illustrate the invention, it being understood that the invention shall not be considered as being limited to them, or by them.

### Example 1

### Synthesis of compound of hydrotalcite type Rh/Mg/Al=0.04/71.00/28.96 (atomic % ratio)

0.1365 parts of Rh(NO₃)₃.2H₂O, by weight, 182 parts of Mg(NO₃)₂.6H₂O, by weight, and 108.6 parts of Al(NO₃)₃.9H₂O, by weight, are dissolved, in a beaker, in 1,000 parts of distilled H₂O, by weight, at the temperature of 60°C (solution "A₁").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₁" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end the product is cooled, and a catalyst having the following composition:

Rh_{0.0004}Mg_{0.7100}Al_{0.2896}O_{1.1450}

is obtained.

The precursor (the product dried at 100°C) and the catalyst were characterized by powder X-ray diffractometry. The precursor showed a single-phase hydrotalcite type structure, and the crystal lattice parameters are reported in following Table 1.

The crystallographic parameters of precursor and calcined product are reported in Tables 2 and 3, respectively.

### Example 2

### Synthesis of compound of hydrotalcite type Rh/Mg/Al=1.00/71.00/28.00 (atomic % ratio)

3.250 parts of Rh(NO₃)₃.2H₂O, by weight, 182 parts of Mg(NO₃)₂.6H₂O, by weight, and 10.5 parts of Al(NO₃)₃.9H₂O, by weight, are dissolved, in a beaker, in 1,000 parts of distilled H₂O, by weight, at the temperature of 60°C (solution "A₂").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₂" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end, the product is cooled, and a catalyst having the following composition:

Rh_{0.0100}Mg_{0.7100}Al_{0.280}O_{1.1450}

is obtained.

The precursor (the product dried at 100°C) and the catalyst were characterized by powder X-ray diffractometry. The precursor showed a single-phase hydrotalcite type structure, and the crystal lattice parameters are reported in following Table 4.

The crystallographic parameters of precursor and calcined product are reported in Tables 2 and 3, respectively.

### Example 3

### Synthesis of compound of hydrotalcite type Rh/Mg/Al=5.00/71.00/24.00 (atomic % ratio)

16.25 parts of Rh(NO₃)₃.2H₂O, by weight, 182 parts of Mg(NO₃)₂.6H₂O, by weight, and 90.03 parts of Al(NO₃)₃.9H₂O, by weight, are dissolved, in a beaker, in 1,000 parts of distilled H₂O, by weight, at the temperature of 60°C (solution "A₃").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₃" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end, the product is cooled, and a catalyst having the following composition:

Rh_{0.0500}Mg_{0.7100}Al_{0.2400}O_{1.1450}

is obtained.

The precursor (the product dried at 100°C) and the catalyst were characterized by powder X-ray diffractometry. The precursor showed a single-phase hydrotalcite type structure, and the crystal lattice parameters are-reported in following Table 5.

The crystallographic parameters of precursor and calcined product are reported in Tables 2 and 3, respectively.

### Example 4

### Synthesis of compound of hydrotalcite type Ru/Mg/Al=1.00/71.00/28.00 (atomic % ratio)

2.46 parts of RuCl₃.xH₂O (41% by weight), 182 parts of Mg(NO₃)₂.6H₂O, by weight, and 105 parts of Al(NO₃)₃.9H₂O, by weight, are dissolved, in a beaker, in 1,000 parts of distilled H₂O, by weight, at the temperature of 60°C (solution "A₄").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₄" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end, the product is cooled, and a catalyst having the following composition:

Ru_{0.0100}Mg_{0.7100}Al_{0.2800}O_{1.1450}

is obtained.

The precursor (the product dried at 100°C) and the catalyst were characterized by powder X-ray diffractometry. The precursor showed a single-phase hydrotalcite type structure, and the crystal lattice parameters are reported in following Table 6.

The crystallographic parameters of precursor and calcined product are reported in Tables 2 and 3, respectively.

### Example 5

### Synthesis of compound of hydrotalcite type Ru/Mg/Al=5.00/71.00/24.00 (atomic % ratio)

13.33 parts of RuCl₃.xH₂O, by weight, (41% by weight), 182 parts of Mg(NO₃)₂.6H₂O, by weight, and 105 parts of Al(NO₃)₃.9H₂O, by weight, are dissolved, in a beaker, in 1,000 parts of distilled H₂O, by weight, at the temperature of 60°C (solution "A₅").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₅" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end, the product is cooled, and a catalyst having the following composition:

Ru_{0.0500}Mg_{0.7100}Al_{0.2400}O_{1.1450}

is obtained.

The precursor (the product dried at 100°C) and the catalyst were characterized by powder X-ray diffractometry. The precursor showed a single-phase hydrotalcite type structure, and the crystal lattice parameters are reported in following Table 7.

The crystallographic parameters of precursor and calcined product are reported in Tables 2 and 3, respectively.

### Example 6 (Comparison Example)

### Synthesis of compound of hydrotalcite type Mg/Al=71.00/29.00 (atomic % ratio)

182 parts of Mg(NO₃)₂.6H₂O, by weight, and 108.8 parts of Al(NO₃)₃.9H₂O, by weight, are dissolved, in a beaker, in 1,000 parts of distilled H₂O, by weight, at the temperature of 60°C (solution "A₆").

A second solution is prepared, at the temperature of 60°C, which contains 127.4 parts of Na₂CO₃, by weight, and 1,500 parts of distilled H₂O, by weight. This solution is acidified by adding concentrated HNO₃ until pH 10 is reached (solution "B").

With vigorous stirring, and at the temperature of 60°C, "A₆" solution is slowly added to "B" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, results to be lower than 0.1% by weight (based on sample dried at 100°C).

The so obtained product is dried at 100°C for 24 hours and is calcined at 900°C for 15 hours. At the end, the product is cooled, and a catalyst having the following composition:

Mg_{0.7100}Al_{0.290}O_{1,1450}

is obtained.

The precursor (the product dried at 100°C) and the catalyst were characterized by powder X-ray diffractometry. The precursor showed a single-phase hydrotalcite type structure, and the crystal lattice parameters are reported in following Table 8.

The crystallographic parameters of precursor and calcined product are reported in Tables 2 and 3, respectively. A small decrease in "a" parameter and unit cell volume is observed, as compared to rhodium or ruthenium containing materials, which is due to the smaller size of aluminum as compared to rhodium and ruthenium. This result confirms that rhodium entered inside the interior of hydrotalcite type structure and of the structure of the catalyst.

### Example 7

### Synthesis of compound of hydrotalcite type Rh/Ni/Mg/Al=5.00/10.00//61.00/24.00 (atomic % ratio)

16.25 parts of Rh(NO₃)₃.2H₂O, by weight, 29.1 parts of Ni(NO₃)₂.6H₂O, by weight, 156.4 parts of Mg(NO₃)₂.6H₂O, by weight, and 90 parts of Al(NO₃)₃.9H₂O, by weight, are dissolved, in a beaker, in 1,000 parts of distilled H₂O, by weight, at the temperature of 60°C (solution "A₇").

A second solution is prepared, at the temperature of 60°C, which contains 240.7 parts of NaHCO₃, by weight, and 1,500 parts of distilled H₂O, by weight (solution "C").

With vigorous stirring, and at the temperature of 60°C, "A₇" solution is slowly added to "C" solution, in such a way as to complete the operation within approximately 30 minutes. During the precipitation, the pH value is kept at 10 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, is lower than 0.1% by weight (based on sample dried at 100°C).

The so obtained product is dried at 100°C for 24 hours and is calcined at 900°C for 15 hours. At the end, the product is cooled, and a catalyst having the following composition:

Rh_{0.0500}Ni_{0,1000}Mg_{0.6100}Al_{0.2400}O_{1.1450}

is obtained.

The precursor (the product dried at 100°C) and the catalyst were characterized by powder X-ray diffractometry. The precursor showed a single-phase hydrotalcite type structure, and the crystal lattice parameters are reported in following Table 9.

The crystallographic parameters of precursor and calcined product are reported in Tables 2 and 3, respectively.

### Example 8 (Comparison Example)

### Synthesis of compound of hydrotalcite type Ni/Mg/Al=10.00/61.00/29.00 (atomic % ratio)

29.1 parts of Ni(NO₃)₂.6H₂O, by weight, 156.4 parts of Mg(NO₃)₂.6H₂O, by weight, and 108.8 parts of Al(NO₃)₃.9H₂O, by weight, are dissolved, in a beaker, in 1,000 parts of distilled H₂O, by weight, at the temperature of 60°C (solution "A₈").

A second solution is prepared, at the temperature of 60°C, which contains 240.7 parts of NaHCO₃, by weight, and 1,500 parts of distilled H₂O, by weight (solution "C").

With vigorous stirring, and at the temperature of 60°C, "A₄" solution is slowly added to "C" solution, in such a way as to complete the operation within approximately 30 minutes. During the precipitation, the pH value is kept at 8 by adding 2M NaOH.

Stirring is continued for 30 minutes, with temperature being constantly kept at 60°C. The precipitate is filtered off and the filter cake is washed with distilled water at the temperature of 60°C until nitrates are completely removed and sodium level, expressed as Na₂O, is lower than 0.1% by weight (based on sample dried at 100°C).

The so obtained product is dried at 100°C for 24 hours and is calcined at 900°C for 15 hours. At the end, the product is cooled, and a catalyst having the following composition:

Ni_{0.1000}Mg_{0.6100}Al_{0.2900}O_{1,1450}

is obtained.

The precursor (the product dried at 100°C) and the catalyst were characterized by powder X-ray diffractometry. The precursor showed a single-phase hydrotalcite type structure, and the crystal lattice parameters are reported in following Table 10.

The crystallographic parameters of precursor and calcined product are reported in Tables 2 and 3, respectively. A small decrease in "a" parameter and unit cell volume is observed, as compared to rhodium or ruthenium containing materials, (as partial replacement for aluminum), due to the smaller size of aluminum as compared to rhodium and ruthenium. This result confirms that rhodium entered inside the interior of hydrotalcite type structure and of the structure of the catalyst.

### Example 9

### Partial methane oxidation

After being reduced under an atmosphere consisting of a mixture of N₂ and H₂ at temperatures comprised within the range of from 200 to 1,000°C, preferably of from 500 to 800°C, the catalysts of Examples 1-3 were used in partial methane oxidation tests.

The tests are carried out in a stationary-bed quartz microreactor of 4 mm of diameter, containing 25 mg of catalyst, as granules having a size comprised within the range of from 7.874 to 11.811 openings/cm (20 to 30 mesh). Methane and oxygen are fed to the reactor diluted in helium, with a molar ratio of methane:oxygen of 2:1 and a partial pressure of methane of 0.29.

The test is carried out under atmospheric pressure, at a temperature of 750°C, as measured by means of a thermocouple installed outside the microreactor, into contact with the catalyst mass, and at space velocities, expressed as GHSV (gas hourly space velocity) comprised within the range of 300,000 to 800,000 cc/g.h. The catalytic test was preceded by a thermal reduction treatment at 750°C, in which a gas nitrogen stream containing approximately 30% of hydrogen was flowed through the microreactor.

The reaction product mixture was analyzed by gas chromatography.

The results from the activity tests are reported in following Table 11, wherein "conversion" means the % methane mols converted based on fed methane mols and "selectivity" means the % CO mols formed as compared to converted methane mols.

**Table 1**

| d | 2theta (deg) | Peak (cts) | I/I₀ (%) |
|---|---|---|---|
| 7.6885 | 11.500 | 5,242 | 100 |
| 5.4138 | 16.360 | 38 | 0.73 |
| 3.8374 | 23.160 | 2,746 | 52.38 |
| 3.1532 | 28.280 | 123 | 2.35 |
| 2.5998 | 34.470 | 1,600 | 30.52 |
| 2.3361 | 38.505 | 605 | 11.54 |
| 2.2997 | 39.140 | 671 | 12.80 |
| 1.9737 | 45.945 | 506 | 9.66 |
| 1.6304 | 56.390 | 36 | 0.69 |
| 1.5262 | 60.625 | 1,232 | 23.50 |
| 1.4995 | 61.820 | 1,011 | 19.29 |
| 1.4946 | 62.045 | 1,011 | 19.29 |
| 1.4175 | 65.835 | 166 | 3.17 |
| 1.3169 | 71.595 | 88 | 1.69 |
| 1.2744 | 74.375 | 151 | 2.89 |

**Table 2**

| Crystallographic parameters | | |
|---|---|---|
| Example | a (A) | Volume (A³) |
| 1 | 3.067(7) | 188.(1) |
| 2 | 3.072(7) | 189.0(9) |
| 3 | 3.075(8) | 191.(1) |
| 4 | 3.064(4) | 188.(1) |
| 5 | 3.067(5) | 189.(1) |
| 6 | 3.063(4) | 188.2(7) |
| 7 | 3.05(1) | 183.(8) |
| 8 | 3.042(7) | 183.(2) |

**Table 3**

| Crystallographic parameters | | | | |
|---|---|---|---|---|
| | Oxide phase | | Spinel phase | |
| Example | a (A) | Volume (A³) | a (A) | Volume (A³) |
| 1 | 4.216(6) | 74.9(2) | 8.094(4) | 530.3(4) |
| 2 | 4.222(5) | 75.3(2) | 8.102(3) | 531.8(3) |
| 3 | 4.220(6) | 75.2(2) | 8.144(4) | 540.2(5) |
| 4 | 4.220(2) | 75.1(1) | 8.101(4) | 531.6(4) |
| 5 | 4.216(2) | 74.9(9) | 8.087(9) | 529.(1) |
| 6 | 4.214(1) | 74.82(5) | 8.077(2) | 526.9(3) |
| 7 | 4.202(1) | 74.20(9) | 8.103(5) | 532.0(6) |
| 8 | 4.207(4) | 74.4(8) | 8.080(5) | 527.5(5) |

**Table 4**

| d | 2theta (deg) | Peak (cts) | I/I₀ (%) |
|---|---|---|---|
| 7.6786 | 11.515 | 4,122 | 100 |
| 3.8489 | 23.090 | 2,016 | 48.91 |
| 3.5018 | 25.415 | 137 | 3.32 |
| 3.1537 | 28.275 | 56 | 1.36 |
| 2.9536 | 30.235 | 79 | 1.92 |
| 2.6079 | 34.360 | 1,414 | 34.30 |
| 2.5889 | 34.620 | 1,608 | 39.01 |
| 2.3420 | 38.405 | 529 | 12.83 |
| 2.3042 | 39.060 | 548 | 13.29 |
| 1.9682 | 46.080 | 396 | 9.61 |
| 1.5277 | 60.560 | 986 | 23.92 |
| 1.5007 | 61.765 | 812 | 19.71 |
| 1.4954 | 62.010 | 729 | 17.69 |
| 1.4190 | 65.755 | 146 | 3.55 |
| 1.3144 | 71.755 | 61 | 1.48 |
| 1.2730 | 74.475 | 108 | 2.62 |

**Table 5**

| d | 2theta (deg) | Peak (cts) | I/I₀ (%) |
|---|---|---|---|
| 7.7287 | 11.440 | 1,815 | 100 |
| 3.9295 | 22.610 | 676 | 37.25 |
| 2.6127 | 34.295 | 718 | 39.58 |
| 2.3449 | 38.355 | 240 | 13.24 |
| 1.9804 | 45.780 | 161 | 8.89 |
| 1.5316 | 60.390 | 625 | 34.44 |
| 1.5007 | 61.765 | 506 | 27.90 |
| 1.4237 | 65.510 | 50 | 2.78 |
| 1.3130 | 71.845 | 49 | 2.70 |
| 1.2707 | 74.630 | 62 | 3.44 |

**Table 6**

| d | 2theta (deg) | Peak (cts) | I/I₀ (%) |
|---|---|---|---|
| 7.6786 | 11.515 | 2,788 | 100 |
| 3.8366 | 23.165 | 1,340 | 48.05 |
| 3.1407 | 28.395 | 56 | 2.02 |
| 2.5911 | 34.590 | 1,260 | 45.21 |
| 2.3096 | 38.463 | 471 | 16.89 |
| 1.5278 | 60.600 | 790 | 28.32 |
| 1.4967 | 61.850 | 692 | 24.81 |
| 1.4208 | 65.660 | 100 | 3.59 |
| 1.3157 | 71.850 | 64 | 2.30 |
| 1.2789 | 74.350 | 79 | 2.84 |

**Table 7**

| d | 2theta (deg) | Peak (cts) | I/I₀ (%) |
|---|---|---|---|
| 7.6952 | 11.490 | 1,260 | 100 |
| 3.8654 | 22.990 | 488 | 38.76 |
| 2.5734 | 34.750 | 620 | 49.20 |
| 2.3162 | 38.850 | 237 | 18.82 |
| 1.9899 | 46.100 | 156 | 12.40 |
| 1.5315 | 60.450 | 480 | 38.06 |
| 1.5011 | 61.600 | 361 | 28.65 |
| 1.4284 | 65.600 | 52 | 4.11 |
| 1.3160 | 71.950 | 34 | 2.67 |

**Table 8**

| d | 2theta (deg) | Peak (cts) | I/I₀ (%) |
|---|---|---|---|
| 7.7355 | 11.430 | 5,776 | 100 |
| 4.5544 | 19.475 | 45 | 0.78 |
| 3.8464 | 23.105 | 2,981 | 51.61 |
| 3.1691 | 28.135 | 135 | 2.33 |
| 2.6020 | 34.440 | 1,936 | 33.52 |
| 2.5827 | 34.705 | 2,125 | 36.79 |
| 2.2955 | 39.215 | 790 | 13.67 |
| 1.9678 | 46.090 | 671 | 11.61 |
| 1.7387 | 52.595 | 36 | 0.62 |
| 1.6459 | 55.810 | 44 | 0.75 |
| 1.5269 | 60.595 | 1,253 | 21.70 |
| 1.5017 | 61.720 | 930 | 16.11 |
| 1.4969 | 61.940 | 1,096 | 18.97 |
| 1.4164 | 65.890 | 174 | 3.02 |
| 1.3139 | 71.785 | 94 | 1.63 |
| 1.2714 | 74.580 | 169 | 2.93 |

**Table 9**

| d | 2theta (deg) | Peak (cts) | I/I₀ (%) |
|---|---|---|---|
| 7.4279 | 11.800 | 1,332 | 100 |
| 3.8219 | 23.255 | 400 | 30.02 |
| 2.5745 | 35.000 | 480 | 36.00 |
| 2.0255 | 46.400 | 90 | 6.77 |
| 1.5223 | 60.975 | 328 | 24.59 |
| 1.3092 | 72.085 | 45 | 3.37 |

**Table 10**

| d | 2theta (deg) | Peak (cts) | I/I₀ (%) |
|---|---|---|---|
| 7.6952 | 11.490 | 5,242 | 100 |
| 3.7954 | 23.420 | 2,663 | 50.80 |
| 3.1235 | 28.555 | 69 | 1.31 |
| 2.5745 | 34.820 | 2,007 | 38.29 |
| 2.2857 | 39.390 | 790 | 15.06 |
| 1.9528 | 46.465 | 586 | 11.17 |
| 1.9223 | 47.245 | 615 | 11.73 |
| 1.7344 | 52.735 | 64 | 1.22 |
| 1.6179 | 56.865 | 27 | 0.52 |
| 1.5183 | 60.975 | 1,156 | 22.05 |
| 1.4916 | 62.185 | 936 | 17.86 |
| 1.4876 | 62.370 | 924 | 17.63 |
| 1.4120 | 66.125 | 166 | 3.17 |
| 1.3626 | 68.850 | 59 | 1.13 |
| 1.3324 | 70.640 | 29 | 0.56 |
| 1.3157 | 71.670 | 92 | 1.76 |
| 1.3061 | 72.280 | 74 | 1.41 |
| 1.2646 | 75.050 | 180 | 3.43 |
| 1.2024 | 79.680 | 125 | 2.39 |

**Table 11**

| Example | GHSV (mL/g.h) | CH₄ conversion (%) | Selectivity to CO (%) |
|---|---|---|---|
| 1 | 300,000 | 73 | 88.1 |
| | 500,000 | 73.3 | 89.9 |
| | 800,000 | 72.9 | 90.9 |
| 2 | 300,000 | 89.7 | 84.6 |
| | 500,000 | 87.3 | 86.5 |
| | 800,000 | 88.4 | 87.2 |
| 3 | 300,000 | 91 | 93.2 |
| | 500,000 | 91 | 93.2 |
| | 800,000 | 89.3 | 93.9 |

## Claims (Claims for the following Contracting State(s): DE, FR, GB, NL)

1. Material having a layered structure of hydrotalcite type, **characterized in that** rhodium and/or ruthenium are contained inside the interior of the structure having the following general formula:
[RhₐRu_{b}X_{c}Y_{d}(OH)₂]^{z+}(Aⁿ⁻ _{z/n}).mH₂O,
wherein
X is a divalent or univalent metal cation,
Y is a trivalent or tetravalent metal cation,
0≤a≤0.5,
0≤b≤0.5,
0.5≤c≤0.9,
0≤d≤0.5,
a+b+c+d=1,
m is 0, or represents a positive integer,
A can be a hydroxyl, any inorganic, organic anion, iso- or hetero-polyanion, anionic complex or organometallic complex, having "n" electrical charge,
z is the total electrical charge of the cationic component with the exclusion of the value φ for the addition a+b and of the value X=CO when b=φ and Y=Al and of the value c=6/7 when a+d = 1/7 and Y= Al and is a divalent metal cation.

2. Material according to claim 1, in which:
0≤a≤0.33,
0≤b≤0.33,
0.66≤c≤0.8,
0≤d≤0.33
with the exclusion of the value φ for the addition a+b and of the value X=CO when b=φ and Y=Al and of the value c=6/7 when a+d = 1/7 and Y= Al and is a divalent metal cation.

3. Material according to claim 1 or 2 in which the divalent metal cation is selected from Mg, Ni, Co, Zn, Fe, Mn, Cu, Ca and Cd.

4. Material according to claim 1 or 2 in which the univalent metal cation is lithium.

5. Material according to claim 1 or 2 in which the trivalent metal cation is selected from Al, Ga, Ni, Co, Fe, Mn, Cr, V, Ti, and In.

6. Material according to claim 1 or 2, in which the tetravalent metal cation is titanium.

7. Use of the material according to any of the preceding claims 1-6 for the reactions of partial methane oxidation to yield synthesis gas.

8. Use of the material according to any of the preceding claims 1-6 for the reactions of nitrogen oxides reduction.

9. Use of the material according to any of the preceding claims 1-6 for hydroformylation reactions.

10. Use of the material according to any of the preceding claims 1-6 for the reactions of hydrogenation of CO, CO₂, or mixtures thereof.

11. Use of the material according to any of the preceding claims 1-6 for water gas shift reactions.

12. Use of the material according to any of the preceding claims 1-6 for hydrogenation reactions of organic substrates in liquid or vapour phase.

13. Process for the partial catalytic oxidation of natural gas in order to obtain synthesis gas, in which the reaction is carried out by operating with a catalyst constituted by the material according to any of the preceding claims from 1 to 6, as a stationary bed, to which a gas stream is fed which contains methane and oxygen, possibly diluted with inert gas, with a molar ratio of methane to oxygen, CH₄:O₂, higher than 1.5, under pressure values comprised within the range of from 50.6 KPa (0.5 atm) to 5066.2 KPa (50 atm), at temperatures comprised within the range of from 300 to 1,000°C and space velocities comprised within the range of from 20,000 to 1,500,000 h⁻¹.

14. Process according to claim 13, in which the pressures are comprised within the range of from 101.3 KPa (1 atm) to 4053 KPa (40 atm), the temperatures are comprised within the range of from 350 to 950°C, and the space velocities are comprised within the range of from 100,000 to 800,000 h⁻¹.

## Claims (Claims for the following Contracting State(s): CH, DK, LI)

1. Material having a layered structure of hydrotalcite type, **characterized in that** rhodium and/or ruthenium are contained inside the interior of the structure having the following general formula:
[RhₐRu_{b}X_{c}Y_{d}(OH)₂]^{z+}(Aⁿ⁻ _{z/n}).mH₂O,
wherein
X is a divalent or univalent metal cation,
Y is a trivalent or tetravalent metal cation,
0≤a≤0.5,
0≤b≤0.5,
0.5≤c≤0.9,
0≤d≤0.5,
a+b+c+d=1,
m is 0, or represents a positive integer,
A can be a hydroxyyl, any inorganic, organic anion, iso- or hetero-polyanion, anionic complex or organometallic complex, having "n" electrical charge,
z is the total electrical charge of the cationic component with the exclusion of the value φ for the addition a+b.

2. Material according to claim 1, in which:
0≤a≤0.33,
0≤b≤0.33,
0.66≤c≤0.8,
0≤d≤0.33
with the exclusion of the value φ for the addition a+b.

3. Material according to claim 1 or 2 in which the divalent metal cation is selected from Mg, Ni, Co, Zn, Fe, Mn, Cu, Ca and Cd.

4. Material according to claim 1 or 2 in which the univalent metal cation is lithium.

5. Material according to claim 1 or 2 in which the trivalent metal cation is selected from Al, Ga, Ni, Co, Fe, Mn, Cr, V, Ti, and In.

6. Material according to claim 1 or 2, in which the tetravalent metal cation is titanium.

7. Use of the material according to any of the preceding claims 1-6 for the reactions of partial methane oxidation to , yield synthesis gas.

8. Use of the material according to any of the preceding claims 1-6 for the reactions of nitrogen oxides reduction.

9. Use of the material according to any of the preceding claims 1-6 for hydroformylation reactions.

10. Use of the material according to any of the preceding claims 1-6 for the reactions of hydrogenation of CO, CO₂, or mixtures thereof.

11. Use of the material according to any of the preceding claims 1-6 for water gas shift reactions.

12. Use of the material according to any of the preceding claims 1-6 for hydrogenation reactions of organic substrates in liquid or vapour phase.

13. Process for the partial catalytic oxidation of natural gas in order to obtain synthesis gas, in which the reaction is carried out by operating with a catalyst constituted by the material according to any of the preceding claims from 1 to 6, as a stationary bed, to which a gas stream is fed which contains methane and oxygen, possibly diluted with inert gas, with a molar ratio of methane to oxygen, CH₄:O₂, higher than 1.5, under pressure values comprised within the range of from 50.6 KPa (0.5 atm) to 5066.2 KPa (50 atm), at temperatures comprised within the range of from 300 to 1,000°C and space velocities comprised within the range of from 20,000 to 1,500,000 h⁻¹.

14. Process according to claim 13, in which the pressures are comprised within the range of from 101.3 KPa (1 atm) to 4053 KPa (40 atm), the temperatures are comprised within the range of from 350 to 950°C, and the space velocities are comprised within the range of from 100,000 to 800,000 h⁻¹.

## Patentansprüche (Patentansprüche für folgende(n) Vertragsstaat(en): DE, FR, GB, NL)

1. Material mit einer Schichtstruktur des Hydrotalcit-Typs, **dadurch gekennzeichnet, dass** Rhodium und/oder Ruthenium im Inneren der Struktur enthalten sind, welche die folgende allgemeine Formel hat
[RhₐRu_{b}X_{c}Y_{d}(OH)₂]^{z+}(Aⁿ⁻ _{z/n}).mH₂O,
worin
X ein zweiwertiges oder einwertiges Metallkation ist,
Y ein dreiwertiges oder vierwertiges Metallkation ist,
0≤a≤0,5,
0≤b≤0,5,
0,5≤c≤0,9,
0≤d≤0,5,
a+b+c+d=1,
m 0 ist oder eine positive ganze Zahl darstellt,
A eine Hydroxylgruppe, irgendein anorganisches oder organisches Anion, Iso- oder Hetero-polyanion, ein anionischer Komplex oder ein Organometallkomplex mit der elektrischen Ladung "n" sein kann,
z die gesamte elektrische Ladung der kationischen Komponente ist,
mit der Ausnahme des Wertes Ø für die Summe a+b und des Wertes X=CO, wenn b=⌀ und Y=Al und des Wertes c=6/7, wenn a+d=1/7 und Y=Al und X = ein zweiwertiges Metallkation.

2. Material nach Anspruch 1, wobei
0≤a≤0,33,
0≤b≤0,33,
0,66≤c≤0,8,
0≤d≤0,33
mit der Ausnahme des Wertes Ø für die Summe a+b und des Wertes X=CO, wenn b=Ø und Y=Al und des Wertes c=6/7, wenn a+d=1/7 und Y=Al und X = ein zweiwertiges Metallkation.

3. Material nach Anspruch 1 oder 2, in dem das zweiwertige Metallkation unter Mg, Ni, Co, Zn, Fe, Mn, Cu, Ca und Cd ausgewählt ist.

4. Material nach Anspruch 1 oder 2, in dem das einwertige Metallkation Lithium ist.

5. Material nach Anspruch 1 oder 2, in dem das dreiwertige Metallkation unter Al, Ga, Ni, Co, Fe, Mn, Cr, V, Ti und In ausgewählt ist.

6. Material nach Anspruch 1 oder 2, in dem das vierwertige Metallkation Titan ist.

7. Verwendung des Materials nach einem der vorhergehenden Ansprüche 1 bis 6 für Reaktionen der partiellen Oxidation von Methan unter Bildung von Synthesegas.

8. Verwendung des Materials nach einem der vorhergehenden Ansprüche 1 bis 6 für die Reaktionen der Reduktion von Stickstoffoxiden.

9. Verwendung des Materials nach einem der vorhergehenden Ansprüche 1 bis 6 für Hydroformylierungsreaktionen.

10. Verwendung des Materials nach einem der vorhergehenden Ansprüche 1 bis 6 für die Reaktionen der Hydrierung von CO, CO₂ oder Gemischen dieser.

11. Verwendung des Materials nach einem der vorhergehenden Ansprüche 1 bis 6 für Wassergas-Umwandlungs(shift)-Reaktionen.

12. Verwendung des Materials nach einem der vorhergehenden Ansprüche 1 bis 6 für Reaktionen der Hydrierung von organischen Substraten in der flüssigen oder der Dampfphase.

13. Verfahren zur partiellen katalytischen Oxidation von Naturgas zur Herstellung von Synthesegas, wobei die Reaktion unter Einsatz eines Katalysators, der aus dem Material nach einem der vorhergehenden Ansprüche 1 bis 6 gebildet ist, als stationäres Bett, dem ein Gasstrom zugeleitet wird, der Methan und Sauerstoff, gegebenenfalls verdünnt mit einem Inertgas enthält, wobei das Molverhältnis von Methan zu Sauerstoff CH₄:O₂ höher als 1,5 ist, unter Drücken im Bereich von 50,6 KPa (0,5 atm) bis 5066,2 KPa (50 atm) bei Temperaturen im Bereich von 300 bis 1000 °C und bei Raumgeschwindigkeiten im Bereich von 20.000 bis 1.500.000 h⁻¹ durchgeführt wird.

14. Verfahren nach Anspruch 13, bei dem die Drücke im Bereich von 101,3 KPa (1 atm) bis 4.053 KPa (40 atm) liegen, die Temperaturen im Bereich von 350 bis 950 C liegen und die Raumgeschwindigkeiten im Bereich von 100.000 bis 800.000 h⁻¹ liegen.

## Patentansprüche (Patentansprüche für folgende(n) Vertragsstaat(en): CH, DK, LI)

1. Material mit einer Schichtstruktur des Hydrotalcit-Typs, **dadurch gekennzeichnet, dass** Rhodium und/oder Ruthenium im Inneren der Struktur enthalten sind, welche die folgende allgemeine Formel hat
[RhₐRu_{b}X_{c}Y_{d}(OH)₂]^{z+}(Aⁿ⁻ _{z/n}).mH₂O,
worin
X ein zweiwertiges oder einwertiges Metallkation ist,
Y ein dreiwertiges oder vierwertiges Metallkation ist,
0≤a≤0,5,
0≤b≤0,5,
0,5≤c≤0,9,
0≤d≤0,5,
a+b+c+d=1,
m 0 ist oder eine positive ganze Zahl darstellt,
A eine Hydroxylgruppe, irgendein anorganisches oder organisches Anion, Iso- oder Hetero-polyanion, ein anionischer Komplex oder ein Organometallkomplex sein kann, mit der elektrischen Ladung "n",
z die gesamte elektrische Ladung der kationischen Komponente ist
mit der Ausnahme des Wertes Ø für die Summe a+b.

2. Material nach Anspruch 1, wobei
0≤a≤0,33,
0≤b≤0,33,
0,66≤c≤0,8,
0≤d≤0,33
mit der Ausnahme des Wertes Ø für die Summe a+b.

3. Material nach Anspruch 1 oder 2, in dem das zweiwertige Metallkation unter Mg, Ni, Co, Zn, Fe, Mn, Cu, Ca und cd ausgewählt ist.

4. Material nach Anspruch 1 oder 2, in dem das einwertige Metallkation Lithium ist.

5. Material nach Anspruch 1 oder 2, in dem das dreiwertige Metallkation unter Al, Ga, Ni, Co, Fe, Mn, Cr, V, Ti und In ausgewählt ist.

6. Material nach Anspruch 1 oder 2, in dem das vierwertige Metallkation Titan ist.

7. Verwendung des Materials nach einem der vorhergehenden Anspruche 1 bis 6 für Reaktionen der partiellen Oxidation von Methan unter Bildung von Synthesegas.

8. Verwendung des Materials nach einem der vorhergehenden Ansprüche 1 bis 6 für die Reaktionen der Reduktion von Stickstoffoxiden.

9. Verwendung des Materials nach einem der vorhergehenden Ansprüche 1 bis 6 für Hydroformylierungsreaktionen.

10. Verwendung des Materials nach einem der vorhergehenden Ansprüche 1 bis 6 für die Reaktionen der Hydrierung von CO, CO₂ oder Gemischen dieser.

11. Verwendung des Materials nach einem der vorhergehenden Ansprüche 1 bis 6 für Wassergas-Umwandlungs(shift)-Reaktionen.

12. Verwendung des Materials nach einem der vorhergehenden Ansprüche 1 bis 6 für Reaktionen der Hydrierung von organischen Substraten in der flüssigen oder der Dampfphase.

13. Verfahren zur partiellen katalytischen Oxidation von Naturgas zur Herstellung von synthesegas, wobei die Reaktion unter Einsatz eines Katalysators, der aus dem Material nach einem der vorhergehenden Ansprüche 1 bis 6 gebildet ist, als stationäres Bett durchgeführt wird, dem ein Gasstrom zugeleitet wird, der Methan und Sauerstoff, gegebenenfalls verdünnt mit einem Inertgas enthält, wobei das Molverhältnis von Methan zu Sauerstoff CH₄:O₂ höher ist als 1,5 unter Drücken im Bereich von 50,6 KPa (0,5 atm) bis 5066,2 KPa (50 atm) bei Temperaturen im Bereich von 300 bis 1000 °C und bei Raumgeschwindigkeiten im Bereich von 20.000 bis 1.500.000 h⁻¹ durchgeführt wird.

14. Verfahren nach Anspruch 13, bei dem die Drücke im Bereich von 101,3 KPa (1 atm) bis 4.053 KPa (40 atm) liegen, die Temperaturen im Bereich von 350 bis 950 C liegen und die Raumgeschwindigkeiten im Bereich von 100.000 bis 800,000 h⁻¹ liegen.

## Revendications (Revendications pour l'(les) Etat(s) contractant(s) suivant(s): DE, FR, GB, NL)

1. Matériau ayant une structure en couche de type hydrotalcite, **caractérisé en ce que** le rhodium et/ou le ruthénium sont contenus à l'intérieur de la structure ayant la formule générale suivante :
[RhₐRu_{b}X_{c}Y_{d}(OH)₂]^{z+}(Aⁿ⁻ _{z/n}).mH₂O,
dans laquelle
X est un cation métallique divalent ou univalent,
Y est un cation métallique trivalent ou tétravalent,
0≤a≤0,5,
0≤b≤0,5,
0,5≤c≤0,9,
0≤d≤0,5,
a+b+c+d=1,
m est 0 ou représente un nombre entier positif,
A peut être un hydroxyle, tout anion minéral, organique, un iso- ou hétéro-polyanion, un complexe anionique ou un complexe organométallique ayant une charge électrique "n",
z est la charge électrique totale du composant cationique avec l'exclusion de la valeur φ pour l'addition a+b
et de la valeur X=CO lorsque b=φ et Y=Al
et de la valeur C=6/7 lorsque a+d=1/7 et Y=Al
et X est un cation métallique divalent.

2. Matériau selon la revendication 1, dans lequel :
0≤a≤0,33,
0≤b≤0,33,
0,66≤c≤0,8,
0≤d≤0,33
avec l'exclusion de la valeur φ pour l'addition a+b
et de la valeur X=CO lorsque b=φ et Y=Al
et de la valeur C=6/7 lorsque a+d=1/7 et Y=Al
et X est un cation métallique divalent.

3. Matériau selon la revendication 1 ou 2, dans lequel le cation métallique divalent est choisi à partir de Mg, Ni, Co, Zn, Fe, Mn, Cu, Ca et Cd.

4. Matériau selon la revendication 1 ou 2, dans lequel le cation métallique univalent est le lithium.

5. Matériau selon la revendication 1 ou 2, dans lequel le cation métallique trivalent est choisi à partir de Al, Ga, Ni, Co, Fe, Mn, Cr, V, Ti et In.

6. Matériau selon la revendication 1 ou 2, dans lequel le cation métallique tétravalent est le titane.

7. Utilisation du matériau selon l'une quelconque des revendications 1-6 pour les réactions d'oxydation partielle de méthane pour donner du gaz de synthèse.

8. Utilisation du matériau selon l'une quelconque des revendications précédentes 1-6 pour les réactions de la réduction des oxydes d'azote.

9. Utilisation du matériau selon l'une quelconque des revendications précédentes 1-6 pour les réactions d'hydroformylation.

10. Utilisation du matériau selon l'une quelconque ; des revendications précédentes 1-6 pour les réactions d'hydrogénation de CO, CO₂ ou leurs mélanges.

11. Utilisation du matériau selon l'une quelconque des revendications précédentes 1-6 pour les réactions de décalage au gaz.

12. Utilisation du matériau selon l'une quelconque des revendications précédentes 1-6 pour les réactions d'hydrogénation de substrats minéraux en phase liquide ou vapeur.

13. Procédé pour l'oxydation catalytique partielle de gaz naturel pour obtenir le gaz de synthèse, dans lequel la réaction est conduite en travaillant avec un catalyseur constitué par le matériau selon l'une quelconque des revendications précédantes de 1 à 6, comme lit stationnaire, auquel un courant gazeux est alimenté qui contient du méthane et de l'oxygène, éventuellement dilué avec du gaz inerte, avec un rapport molaire entre le méthane et l'oxygène, CH₄:O₂, supérieur à 1,5, sous des valeurs de pression comprises dans la plage allant de 50,6 KPa (0,5 atm) à 5066,2 KPa (50 atm), à une température comprise dans la plage allant de 300 à 1000°C et des vitesses spatiales comprises dans la plage allant de 20 000 à 1 500 000 h⁻¹.

14. Procédé selon la revendication 13, dans lequel les pressions sont comprises dans la plage allant de 101,3 KPa (1 atm) à 4053 KPa (40 atm), les températures sont comprises dans la plage de 350 à 950°C, et les vitesses spatiales sont comprises dans la plage allant de 100 000 à 800 000 h⁻¹.

## Revendications (Revendications pour l'(les) Etat(s) contractant(s) suivant(s): CH, DK, LI)

1. Matériau ayant une structure en couche de type hydrotalcite, **caractérisé en ce que** le rhodium et/ou le ruthénium sont contenus à l'intérieur de la structure ayant la formule générale suivante :
[RhₐRu_{b}X_{c}Y_{d}(OH)₂]^{z+}(Aⁿ⁻ _{z/n}).mH₂O,
dans laquelle
X est un cation métallique divalent ou univalent,
Y est un cation métallique trivalent ou tétravalent,
0≤a≤0,5,
0≤b≤0,5,
0,5≤c≤0,9,
0≤d≤0,5,
a+b+c+d=1,
m est 0 ou représante un nombre entier positif,
A peut être un hydroxyle, tout anion minéral, organique, un iso- ou hétéro-polyanion, un complexe anionique ou un complexe organométallique, ayant une charge électrique "n",
z est la charge électrique totale du composant cationique à l'exclusion de la valeur φ pour l'addition a+b.

2. Matériau selon la revendication 1, dans lequel :
0≤a≤0,33,
0≤b≤0,33,
0,66≤c≤0,8,
0≤d≤0,33
à l'exclusion de la valeur φ pour l'addition a+b.

3. Matériau selon la revendication 1 ou 2, dans lequel le cation métallique divalent est choisi à partir de Mg, Ni, Co, Zn, Fe, Mn, Cu, Ca et Cd.

4. Matériau selon la revendication 1 ou 2, dans lequel le cation métallique univalent est le lithium.

5. Matériau selon la revendication 1 ou 2, dans lequel le cation métallique trivalent est choisi à partir de Al, Ga, Ni, Co, Fe, Mn, Cr, V, Ti et In.

6. Matériau selon la revendication 1 ou 2, dans lequel le cation métallique tétravalent est le titane.

7. Utilisation du matériau selon l'une quelconque des revendications 1-6 pour les réactions d'oxydation partielle de méthane pour donner du gaz de synthèse.

8. Utilisation du matériau selon l'une quelconque des revendications précédentes 1-6 pour les réactions de la réduction des oxydes d'azote.

9. Utilisation du matériau selon l'une quelconque des revendications précédentes 1-6 pour les réactions d'hydroformylation.

10. Utilisation du matériau selon l'une quelconque des revendications précédentes 1-6 pour les réactions d'hydrogénation de CO, CO₂ ou leurs mélanges.

11. Utilisation du matériau selon l'une quelconque des revendications précédentes 1-6 pour les réactions de décalage au gaz.

12. Utilisation du matériau selon l'une quelconque des revendications précédentes 1-6 pour les réactions d'hydrogénation de substrats minéraux en phase liquide ou vapeur.

13. Procédé pour l'oxydation catalytique partielle de gaz naturel pour obtenir le gaz de synthèse, dans lequel la réaction est conduite en travaillant avec un catalyseur constitué par le matériau selon l'une quelconque des revendications précédentes de 1 à 6, comme lit stationnaire, auquel un courant gazeux est alimenté qui contient du méthane et de l'oxygène, éventuellement dilué avec du gaz inerte, avec un rapport molaire entre le méthane et l'oxygène, CH₄:O₂, supérieur à 1,5, sous des valeurs de pression comprises dans la plage allant de 50,6 KPa (0,5 atm) à 5066,2 KPa (50 atm), à une température comprise dans la plage allant de 300 à 1000°C et des vitesses spatiales comprises dans la plage allant de 20 000 à 1 500 000 h⁻¹.

14. Procédé selon la revendication 13, dans lequel les pressions sont comprises dans la plage allant de 101,3 KPa (1 atm) à 4053 KPa (40 atm), les températures sont comprises dans la plage de 350 à 950°C, et les vitesses spatiales sont comprises dans la plage allant de 100 000 à 800 000 h⁻¹.
